# EUROPEAN PATENT APPLICATION

(11) **EP 1 491 637 A1**
(43) Date of publication of application: **29.12.2004**
(21) Application number: 03291540.7
(22) Date of filing: 24.06.2003
(51) Int. Cl.: C12Q 1/68

(54) **Process for improving efficiency of DNA amplification reactions**

(71) Applicant: Nichirei Corporation, Tokyo 104-8402 (JP)
(72) Inventor: Koizumi, Takeshi, Tokyo (JP); Hamano, Yoko, Chiba-shi, Chiba-ken (JP); Yamamoto, Satoshi, Chiba-shi, Chiba-ken (JP)
(74) Representative: Hubert, Philippe

(57) **Abstract**

The present invention has an object of providing a process for improving the efficiency of a DNA amplification reaction, and a process for improving the hybridization specificity of an oligonucleotide to a DNA.

The present invention provides a process for improving the efficiency of a DNA amplification reaction, wherein a primer in which a compound such as LC-Red 705 or an oligonucleotide with a GC content of at least 25% and with at least four bases is added to the 5' terminus is used as the primer; as well as a process for improving the hybridization specificity of an oligonucleotide to a DNA sample, wherein an oligonucleotide in which a compound such as LC-Red 705 is conjugated to the 5' terminus is used for hybridizing to a DNA.

## Description

### BACKGROUND OF THE INVENTION

### Field of the Invention

The present invention relates to a process for improving the efficiency of a DNA amplification reaction, and a process for improving the hybridization specificity of an oligonucleotide to a DNA sample.

### Description of Related Art

DNA amplification is extremely important in the detection of genes, and within the field of DNA amplification, the PCR method enables a large amplification of a targeted portion of nucleotide sequences within the DNA, and is a method which is used not only within biotechnology, but also within a variety of other fields.

However, when a specific detection primer is designed, the primer must include a base position specific to the target sequence.

Unfortunately, sequences including this type of position are frequently unsuitable as primers. In other words, in cases in which, for example, the AT content is extremely high, or the forward and reverse melting temperatures (Tm) do not match, the efficiency of the amplification deteriorates, making the sequence impractical for use as a primer. This problem becomes a considerable drawback in cases in which very small quantities of DNA need to be detected.

Furthermore in the PCR method, in order to improve the amplification efficiency, the amplification optimum temperature conditions need to be determined. However, determining the optimum temperature conditions requires a series of complex preliminary tests.

Accordingly, the present invention has an object of providing a process for simplifying the operation needed for determining the optimum temperature conditions, and improving the efficiency of a DNA amplification reaction.

In addition, the present invention also has an object of providing a process for improving the hybridization specificity of an oligonucleotide to a DNA.

### BRIEF SUMMARY OF THE INVENTION

The inventors of the present invention discovered an extremely surprising fact. Namely, when an artificial non-specific sequence is added to the 5' terminus of a degenerate primer, then the PCR amplification efficiency increased, and when the sequence added to the 5' terminus is removed, the PCR amplification efficiency decreased.

Consequently, the inventors conducted further research and discovered, quite unexpectedly, that by not only adding an artificial non-specific sequence, but also conjugating even a compound such as LC-Red 705, to the 5' terminus, the PCR amplification efficiency could be improved, resulting in an improvement in the efficiency of the DNA amplification reaction, and the inventors were hence able to complete the present invention. The optimum temperature range for annealing could be widened, meaning the preliminary tests for investigating the annealing conditions could be simplified, the overall process could be simplified considerably.

In other words, a first aspect of the present invention provides a process for improving the efficiency of a DNA amplification reaction, wherein a primer in which a compound selected from a group consisting of LC-Red 705, an amino group, a phosphate group, biotin, DIG, DNP, TAMRA, Texas-Red, ROX, XRITC, rhodamine, LC-Red 640, a mercapto group, psoralen, cholesterol, FITC, 6-FAM, TET, cy3, cy5, BODIPY 564/570, BODIPY 500/510, BODIPY 530/550, BODIPY 581/591 (hereafter described as the "specified compounds group") and oligonucleotides with a GC content of at least 25% and with at least four bases (hereafter described as the "specified bases") is added to the 5' terminus is used as a primer.

A second aspect of the present invention provides a process for improving the hybridization specificity of an oligonucleotide to a DNA sample, wherein an oligonucleotide in which a compound selected from the above specified compounds group is conjugated to the 5' terminus is used for hybridizing to the DNA.

### BRIEF DESCRIPTION OF THE DRAWINGS

FIG. 1 is a graph showing the relationship between the number of PCR cycles and the fluorescence intensity in annealing conditions of 0 seconds at 60°C.
FIG. 2 is a graph showing the relationship between the number of PCR cycles and the fluorescence intensity in annealing conditions of 5 seconds at 60°C.
FIG. 3 is a graph showing the relationship between the number of PCR cycles and the fluorescence intensity in annealing conditions of 10 seconds at 60°C.
FIG. 4 is a graph showing the relationship between the number of PCR cycles and the fluorescence intensity in annealing conditions of 5 seconds at 64°C.

### DETAILED DESCRIPTION OF THE INVENTION

In the first and the second aspects of the present invention, there are no particular restrictions on the primer to which the compound selected from the specified compounds group and the specified bases is conjugated or added, nor on the oligonucleotide to which the compound selected from the specified compounds group is conjugated, provided they represent a primer or an oligonucleotide which is typically used in DNA amplification. Furthermore, because the present invention enables the DNA amplification efficiency to be improved, some primers which are not normally usable for DNA amplification can also be used.

In the first and the second aspects of the present invention, the compounds of the specified compounds group are non-specific with respect to the target sequence to be amplified by DNA amplification. DIG is an abbreviation for digoxigenin, DNP is an abbreviation for dinitrophenyl, TAMRA refers to carboxytetramethylrhodamine, Texas-Red is 1H,5H,11H,15H-Xantheno[2,3,4-ij:5,6,7-i'j']diquinolizin-18-ium, 9-[2 (or 4)-[[[6-(2,5-dioxo-1-pyrrolidinyl)oxy]-6-oxohexyl]amino]sulfonyl]-4 (or 2) sulfophenyl]-2,3,6,7,12,13,16,17-octahydro-, inner salt, ROX is an abbreviation for rhodamine X, XRITC refers to rhodamine X isothiocyanate, FITC is an abbreviation for fluorescein isothiocyanate, 6-FAM refers to 6-carboxyfluorescein, TET is an abbreviation for tetrachlorofluorescein, BODIPY 564/570 is 4,4-difluoro-5-styryl-4-bora-3a,4a-diaza-s-indacene-3-propionic acid, succinimidyl ester, BODIPY 530/550 is 4,4-difluoro-5,7-diphenyl-4-bora-3a,4a-diaza-s-indacene-3-propionic acid, succinimidyl ester, and BODIPY 581/591 is 4,4-difluoro-5-(4-phenyl-1,3-butadienyl)-4-bora-3a,4a-diaza-s-indacene-3-propionic acid, succinimidyl ester.

According to the first aspect of the present invention, the specified bases may be specific or non-specific to the nucleotide sequence to be hybridized. As used herein, being "specific" includes not only a case where an addendum sequence to the primer is complementary to a region of a template which is not contiguous to a region to which the primer hybridizes, but also a case where the addendum sequence to the primer is complementary to a region of a template which is contiguous to a region to which the primer hybridizes (especially a 3' region of the template corresponding to the 5' region of the primer). The latter case is used in the prior art to adjust the Tm values of primers; however, in the present invention, it is used to improve the amplification efficiency.

Being non-specific includes a case where no relation exists between the primer and a template, namely the addendum sequence has a nucleotide sequence in which a double strand is not formed contiguously in GC and AT base pairs. Even under non-specific cases like these, it is possible in the present invention to increase the highest annealing temperature of a primer. Therefore, in the first aspect of the present invention, the specified bases may be specific or non-specific to the nucleotide sequence to be hybridized (template DNA). However, the specified sequence is preferably non-specific in order to increase an amplification efficiency.

In the first aspect of the present invention, either one, or two or more compounds selected from the specified compounds group and the specified bases can be used.

In the second aspect of the present invention, either one, or two or more compounds selected from the specified compounds group can be used.

In the first and the second aspects of the present invention, the oligonucleotide or the primer and the compound selected from the specified compounds group may also be conjugated via a linker. Examples of suitable linkers include hydrocarbon groups of 2 to 16 carbon atoms.

In the first aspect of the present invention, the oligonucleotide added to the 5' terminus of the primer (the addendum sequence) preferably has a high GC content. Specifically, the oligonucleotide must have a GC content of at least 50%, and an addendum sequence of at least four bases is also preferable. The efficiency of the DNA amplification reaction improves with increasing GC content, values of 60 % or greater, 65% or greater, 70% or greater, 75% or greater, 80% or greater, 85% or greater, 90% or greater, 95% or greater, and 100% are even more desirable. Any person skilled in the art can determine the GC content based on the length of the addendum sequence, and non-complimentarity between the addendum sequence and the sequence to be amplified or a primer etc. If the addendum sequence becomes too long, then the efficiency of the DNA amplification reaction may deteriorate, and typically addendum sequences of no more than 40 bases are preferred. Furthermore, for preventing formation of a primer dimmer, the quantity of either G or C preferably accounts for at least 50%, and the quantity of either A or T preferably accounts for at least 50%.

Specific examples of preferred nucleotide sequences are listed below. Furthermore, sequences of 9 or more bases can be generated by suitable combinations of the 2 to 8 nucleotide sequences below. In the sequences below, S represents either C or G, and W represents either A or T. The quantity of either G or C preferably accounts for at least 50%, and the quantity of either A or T preferably accounts for at least 50%.

SS, SW, WS, SSS, SSW, SWS, WSS, SSSS, SSSW, SSWS, SWSS, WSSS, SSSSS, SSSSW, SSSWS, SSWSS, SWSSS, WSSSS, SSSSSS, SSSSSW, SSSSWS, SSSWSS, SSWSSS, SWSSSS, WSSSSS, SSSSSSS, SSSSSSW, SSSSSWS, SSSSWSS, SSSWSSS, SSWSSSS, SWSSSSS, WSSSSSS, SSSSSSSS, SSSSSSSW, SSSSSSWS, SSSSSWSS, SSSSWSSS, SSSWSSSS, SSWSSSSS, SWSSSSSS, WSSSSSSS, SSSSSSWW, SSSSSWSW, SSSSWSSW, SSSWSSSW, SSWSSSSW, SWSSSSSW, WSSSSSSW, SSSSSWWS, SSSSWSWS, SSSWSSWS, SSWSSSWS, SWSSSSWS, WSSSSSWS, SSSSWWSS, SSSWSWSS, SSWSSWSS, SWSSSWSS, WSSSWSS, SSSWWSSS, SSWSWSSS, SWSSWSSS, WSSSWSSS, SSWWSSSS, SWSWSSSS, WSSWSSSS, SWWSSSSS, WSWSSSSS, WWSSSSSS

Furthermore, specific examples of oligonucleotides include those of up to 20 bases formed from repeating units of AGTC, AAGT, GGAC or GGGC.

Furthermore, it is preferable that the addendum sequence has a nucleotide sequence which does not form a secondary structure, thereby hindering an amplification reaction. Specifically, it is preferable that the addendum sequence displays low base pair formation between the 3' terminus sequences, and no base pair formation is even more preferred. The minimum preferred requirements are for no consecutive base pair formation. In addition, the primer with the addendum sequence also preferably displays low base pair formation, and no base pair formation is even more preferred.

In the first aspect of the present invention, the compound selected from the aforementioned specified compounds group or the specified bases can be conjugated or added to the 5' terminus of the primer in accordance with standard methods. In order to conjugate or add two or more different compounds from the specified compounds group or the specified bases, either a compound can be conjugated first, and an oligonucleotide including specified bases subsequently synthesized, or alternatively, an oligonucleotide including specified bases can be synthesized first, and a compound subsequently conjugated. An example of a primer with two or more compounds from the specified compounds group or the specified bases added is FITC with a double repeating sequence of GGGC added.

In the second aspect of the present invention, the compound selected from the aforementioned specified compounds group can be conjugated to the 5' terminus of an oligonucleotide in accordance with standard methods. In order to conjugate two or more different compounds from the specified compounds group or the specified bases, either a compound can be conjugated first, and an oligonucleotide including specified bases subsequently synthesized, or alternatively, an oligonucleotide including specified bases can be synthesized first, and a compound subsequently conjugated.

In the first aspect of the present invention, by conjugating a compound selected from the specified compounds group to a primer or adding the specified bases to a primer, both the annealing speed of the primer to the amplified product and the annealing stability can be improved, meaning the primer is ideally suited to normal PCR. The improvement in the annealing speed and annealing stability can be also observed when the specified bases are non-complementary to the template DNA (see the results shown in Table 2).

In addition, the present invention can also be ideally applied to asymmetric PCR.

Asymmetric PCR is a method used for rapidly amplifying a single strand DNA, such as in cases where a target DNA fragment needs to be directly sequenced. In other words, whereas in normal PCR the concentrations of the pair of primers used are equal, in asymmetric PCR, the concentration of one of the primers is raised to several times, or several dozen times that of the other primer. By so doing, the lower concentration primer is consumed first, and the remaining PCR proceeds only from the residual higher concentration primer, producing a large quantity of the DNA strand corresponding with the higher concentration primer. Furthermore, in thermal asymmetric PCR, which represents one specific type of asymmetric PCR, a pair of primers is used which display a difference in Tm of at least 10°C, and first PCR is conducted under conditions in which the primer with the lower Tm value will also undergo annealing, and subsequently PCR is conducted under conditions in which only the primer with the higher Tm value will undergo annealing.

However, asymmetric PCR suffers from the types of problems described below. Namely, if the concentrations of the template DNA and the primers are not optimized, then the amplification of the single strand is low (Production of Single Stranded DNA by Asymmetric PCR, PCR Protocols, A guide to Methods and Applications, Academic Press, Inc. 1990). However, such optimization requires complex preliminary tests.

Furthermore in thermal asymmetric PCR, a set of specific primers with a large difference in annealing temperature of at least 10°C must be prepared, and this is not necessarily a simple task.

Another method of rapidly amplifying a single strand DNA utilizes the difference in amplification ability within a pair of primers. For example, hybrid primers of DNA and RNA can be used. RNA primers display a weaker contribution to extension reactions than DNA primers, and consequently if PCR is conducted with these types of hybrid primers, then the amplification at the pure DNA side will be larger, yielding a single strand DNA.

However, this method results in a single strand DNA due to the low amplification ability of the RNA side, and does not result from any improvement in the amplification ability of the desired DNA.

In contrast, in the first aspect of the present invention, by conjugating a compound selected from the specified compounds group or adding the specified bases to only one of the pair of primers, a large difference in amplification efficiency can be generated between the two primers, meaning the complex operations of optimizing the concentrations of the template DNA and the primers are not required. Consequently, by applying the first aspect of the present invention to asymmetric PCR, the amplification efficiency for a single strand DNA can be improved markedly. Furthermore, the first aspect of the present invention also enables the optimum temperature range for the primers to be widened, making the invention also applicable to thermal asymmetric PCR.

Conventionally, asymmetric PCR has been conducted by inhibiting the extension of one of the primers, that is, by effectively lowering the overall PCR efficiency. In contrast, the first aspect of the present invention enables asymmetric PCR to be conducted by improving the amplification efficiency of one of the primers. In other words, when compared with conventional PCR, asymmetric PCR using the present invention suffers no reduction in amplification efficiency. Accordingly, the first aspect of the present invention is particularly effective in those cases in which generation of a single strand DNA is required while maintaining a high level of amplification efficiency, such as the case in which a minute quantity of DNA such as a pathogen needs to be detected and typed rapidly and easily.

In addition, the present invention can also be ideally applied to degenerate PCR. In degenerate PCR, a mixture of between several hundred and several thousand different primers are used, meaning that the optimum annealing temperature will differ for each of the sequences within the mixture. As a result, the setting of the amplification temperature is comparatively difficult. However, this type of problem can also be resolved using the present invention. Moreover, because the annealing temperature can be set to a relatively high temperature, non-specific amplification can be suppressed, enabling a more efficient amplification.

A primer to which a compound selected from the specified compounds group of the first aspect of the present invention has been conjugated can also be used as a probe.

In the second aspect of the present invention, by using an oligonucleotide in which a compound selected from the above-specified compounds group has been conjugated to the 5' terminus, the hybridization specificity of the oligonucleotide to DNA can be improved. In other words, in comparison with an oligonucleotide without a specified compound conjugated, an oligonucleotide with a specified compound conjugated offers an improvement in both the speed of hybridization to the DNA, and the hybridization stability.

### EXAMPLES

As follows is a more detailed description of the present invention based on a series of examples. However, the present invention is in no way restricted to the examples presented below.

### Example 1

### Comparison of the Upper Limit Annealing Temperature for Amplification

Using primers for the detection of Vibrio parahaemolyticus with a compound selected from the specified compounds group conjugated to the 5' terminus, and utilizing a PCR Express device manufactured by Hybaid Co., Ltd with a Gradient Block Module added, the upper limit annealing temperature for amplification was measured by conducting PCR under the conditions described below.

The primers for the detection of Vibrio parahaemolyticus utilized a nucleotide sequence represented by the sequence number 1 as the forward side primer and a nucleotide sequence represented by the sequence number 2 as the reverse side primer.
Sequence number 1: aagaagacct agaagatgat
Sequence number 2: gttaccagta atagggca
   Each of the compounds of the specified compounds group shown in Table 1 was conjugated to the 5' termini of the forward side primer and the reverse side primer. In a separate preparation, chromosome DNA extracted from a type strain (IFO12711T) of Vibrio parahaemolyticus was used as a template, and PCR was conducted using a rpoD gene amplification universal primer (refer to Japanese Unexamined Patent Application, Publication No. Hei 8-256798: sequence numbers 3 and 4) to prepare an amplified product. Subsequently, using this amplified product as a template, PCR tests were conducted under a plurality of different annealing temperature conditions, using the aforementioned primers with added compounds from the specified compounds group.
Sequence number 3: yatgmgngar atgggnacng t
   (y stands for a base T or U, or C; m stands for A or C; and n stands for A, C, G, or T or U)
Sequence number 4: ngcytcnacc atytcyttyt t

The PCR conditions were as follows. (1) Activation of Taq polymerase (AmpliTaq Gold, manufactured by Applied Biosystems Co., Ltd.): 10 minutes at 95°C. (2) Denaturation: 1 minute at 94°C. (3) Annealing: 30 seconds at 55.1°C, 55.5°C, 56.3°C, 57.7°C, 59.4°C, 61.4°C, 63.3°C, 65.3°C, 67.6°C, 69.0°C, 69.7°C and 70.2°C. (4) Extension reaction: 1 minute at 72°C. The above steps (2) through (4) were repeated for 40 cycles. (5) Extension reaction: 10 minutes at 72°C. (6) Cooling: cooled to 4°C.

Subsequently, the thus obtained amplified fragments were analyzed by agarose gel electrophoresis, and the upper limit annealing temperatures were determined. A primer with no conjugated compound from the specified compounds group was used as a control. The results for the upper limit annealing temperatures, and the temperature increases in the upper limit annealing temperatures relative to the control value, are shown in Table 1.

**Table 1**

| Conjugated Compound | Linker | Effect | Upper limit annealing temperature | Temperature increase (comparison with control primer) |
|---|---|---|---|---|
| BODIPY564/570 | | AA (Max) | 63.3°C | 5.6°C |
| LC-Red 705 | none | AA | 63.3°C | 5.6°C |
| Amino group C3 | C3 | A | 61.4°C | 3.7°C |
| Phosphate group | none | A | 61.4°C | 3.7°C |
| Biotin | C10 | A | 61.4°C | 3.7°C |
| DIG | C6 | A | 61.4°C | 3.7°C |
| DNP | C14 | A | 61.4°C | 3.7°C |
| TAMRA | C6 | A | 61.4°C | 3.7°C |
| Texas-Red | C6 | A | 61.4°C | 3.7°C |
| ROX | C6 | A | 61.4°C | 3.7°C |
| XRITC (Rhodamine 600) | C6 | A | 61.4°C | 3.7°C |
| Rhodamine | C6 | A | 61.4°C | 3.7°C |
| LC-Red 640 | C6 | A | 61.4°C | 3.7°C |
| BODIPY500/510 | | A | 61.4°C | 3.7°C |
| BODIPY530/550 | | A | 61.4°C | 3.7°C |
| BODIPY581/591 | | A | 61.4°C | 3.7°C |
| Amino group C6 | C6 | B | 59.4°C | 1.7°C |
| SH (thiol) | none | B | 59.4°C | 1.7°C |
| Psoralen C2 | C2 | B | 59.4°C | 1.7°C |
| Psoralen C6 | C6 | B | 59.4°C | 1.7°C |
| Cholesterol | | B | 59.4°C | 1.7°C |
| FITC | C6 | B | 59.4°C | 1.7°C |
| 6-FAM | C6 | B | 59.4°C | 1.7°C |
| TET | C6 | B | 59.4°C | 1.7°C |
| cy3 | C3 | B | 59.4°C | 1.7°C |
| cy5 | C3 | B | 59.4°C | 1.7°C |
| No label (Control) | | - | 57.7°C | - |
| AA: Excellent | | | | |
| A: Good | | | | |
| B: Fair | | | | |

The primers with added compounds from the specified compounds group displayed an increase in the upper limit annealing temperature, and produced an improvement in amplification efficiency.

### Example 2

### Comparison of the Upper Limit Annealing Temperature for Amplification

A nucleotide sequence represented by the sequence number 3 was used as the forward side primer and a nucleotide sequence represented by the sequence number 4 was used as the reverse side primer. A 4mer through to a 20mer, with a nucleotide sequence shown in Table 2 as the repeating unit, was added to the 5' terminus of each of these primers, and the upper limit annealing temperature for amplification was measured by conducting PCR under the same conditions as the example 1, using a PCR Express device manufactured by Hybaid Co., Ltd with a Gradient Block Module added, and using chromosome DNA extracted from a type strain (IFO 12711 T) of Vibrio parahaemolyticus as a template, with an annealing time of 1 minute. A primer with no added oligonucleotide was used as a control. Furthermore, primers containing a 4mer through to a 20mer of a repeating unit of AAAT with a GC content of 0% were used for the purposes of comparison. The results for the upper limit annealing temperatures, and the temperature increases in the upper limit annealing temperatures relative to the control value (shown in brackets) are shown in Table 2.

**Table 2.**

| | Repeating Sequence | | | | | |
|---|---|---|---|---|---|---|
| | AGTC | AAGT | GGAC | GGGC | AAAT | None |
| x1 (4mer) | 63.3 (+1.9) | 63.3 (+1.9) | 65.3 (+3.9) | 67.6 (+6.2) | 61.4 (±0) | |
| x2 (8mer) | 63.3 (+1.9) | 63.3 (+1.9) | 65.3 (+3.9) | 69 (+7.6) | 59.4 (-2.0) | |
| x3 (12mer) | 65.3 (+3.9) | 63.3 (+1.9) | 67.6 (+6.2) | 63.3 (+1.9) | 59.4 (-2.0) | |
| x4 (16mer) | 63.3 (+1.9) | 63.3 (+1.9) | 63.3 (+1.9) | N.A. | 57.7 (-3.7) | |
| x5 (20mer) | 65.3 (+3.9) | 63.3 (+1.9) | 57.7 (-3.7) | N.A. | 57.7 (-3.7) | |
| GC% | 50% | 25% | 75% | 100% | 0% | 61.4 |
| N.A. Not Amplified | | | | | | |

Primers containing an added oligonucleotide with a GC content of at least 25% and with at least four bases displayed an improvement in the amplification efficiency, and this effect was particularly marked for primers containing an added oligonucleotide with a high GC content.

### Example 3

### Investigation of the Amplification Efficiency in Primers containing a Compound Selected from the Specified Compounds Group or the Specified Bases Added to the 5' Terminus

A nucleotide sequence represented by the sequence number 1 was used as the forward side primer and a nucleotide sequence represented by the sequence number 2 was used as the reverse side primer. Either cy3, cy5, biotin, a 12mer with GGAC as the repeating unit, or a 12mer with AAGT as the repeating unit, were added (conjugated) to the 5' termini of the forward side primer and the reverse side primer, and the kinetics of the amplification reaction were analyzed by conducting real time PCR under conditions described below, using a Light Cycler System (manufactured by Roche Diagnostics Co., Ltd.), and using chromosome DNA extracted from a type strain (IFO12711T) of Vibrio parahaemolyticus as a template.

The PCR conditions were as follows. (1) Denaturation: 1.5 minutes at 95°C. (2) Denaturation: 0 seconds at 95°C. (3) Annealing: 0, 5 or 10 seconds at 60°C, or 5 seconds at 64°C. (4) Extension reaction: 15 seconds at 72°C.

The above steps (2) through (4) were repeated for 40 cycles.

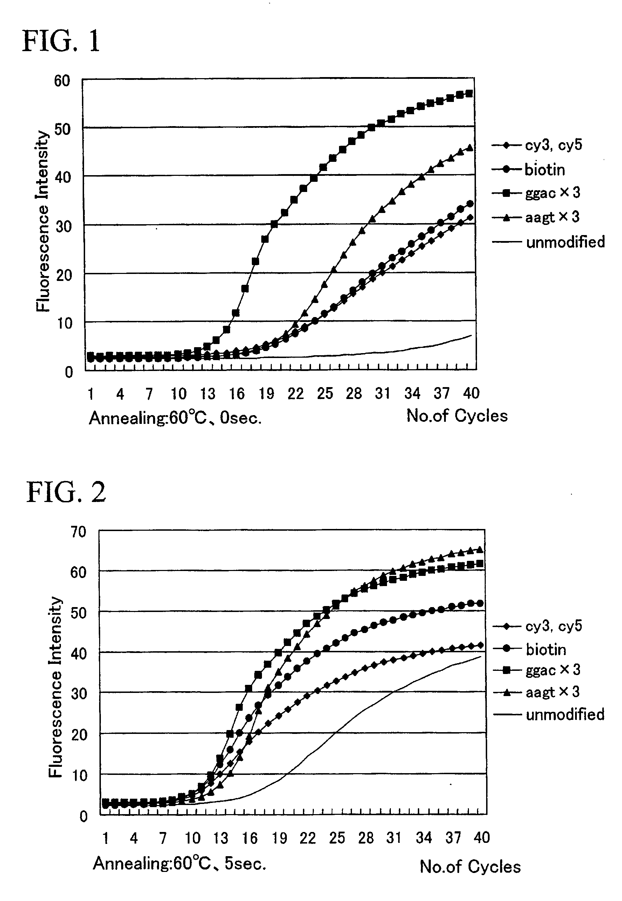

The amplified fragment obtained after each cycle was measured for fluorescence intensity. A primer with no added compound from the specified bases was used as a control. The results are shown in FIG. 1 through FIG. 4. This fluorescence intensity is not derived from the compounds of the specified compounds group conjugated to the primer, but rather is derived from cyber green intercalated to the double strands, and indicates the accumulated quantity of amplified DNA.

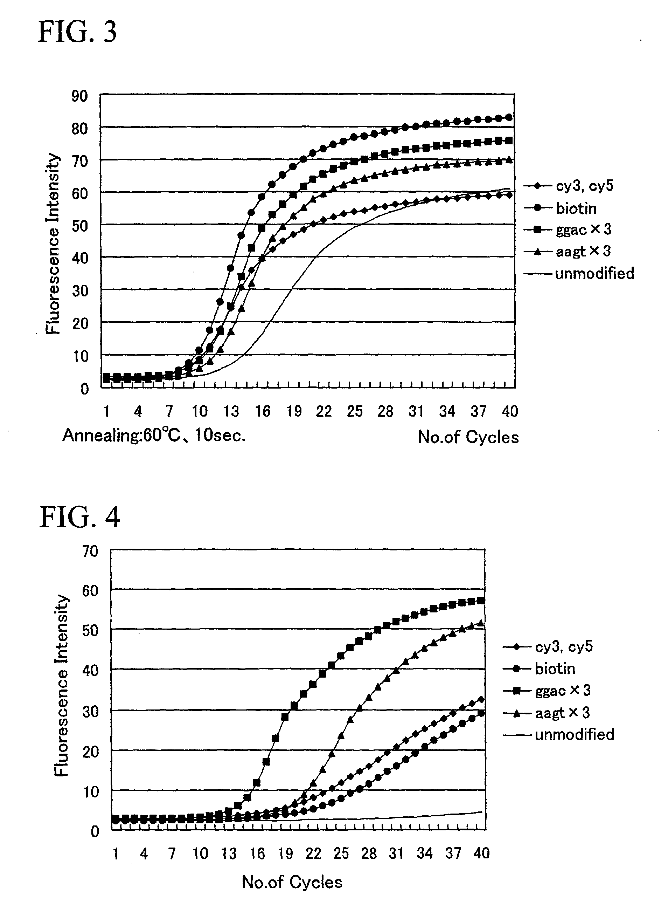

FIG. 1 is a graph showing the relationship between the number of PCR cycles and the fluorescence intensity in annealing conditions of 0 seconds at 60°C. FIG. 2 is a graph showing the relationship between the number of PCR cycles and the fluorescence intensity in annealing conditions of 5 seconds at 60°C. FIG. 3 is a graph showing the relationship between the number of PCR cycles and the fluorescence intensity in annealing conditions of 10 seconds at 60°C. FIG. 4 is a graph showing the relationship between the number of PCR cycles and the fluorescence intensity in annealing conditions of 5 seconds at 64°C.

Under all the amplification conditions, the primers with an added compound selected from the specified compounds group or the specified bases (the modified primers) displayed an earlier rise in the amplification reaction than the primer without an added compound selected from the specified compounds group or the specified bases (the unmodified primer). In other words, when the modified primers are used, the number of cycles required to achieve a constant fluorescence intensity shortens (refer to FIG. 2 and FIG. 3).

If the annealing time is shortened for the same annealing temperature (from 10 seconds (FIG. 3) to 5 seconds (FIG. 2) to 0 seconds (FIG. 1)), then the cycle at which amplification of the unmodified primer commences is delayed considerably (in other words, the amplification weakens). For example, in order to achieve a fluorescence intensity exceeding 10, 14 cycles are required in the case of an annealing time of 10 seconds (FIG. 3), whereas 20 cycles are required in the case of an annealing time of 5 seconds (FIG. 3), and in the case of an annealing time of 0 seconds, the fluorescence intensity does not exceed 10, even after 40 cycles. In contrast, in the case of the modified primers, the delay in the amplification commencement cycle resulting from shortening of the annealing time is considerably less than that observed for the unmodified primer. In other words, when used in a detection system, the modified primers provide an increase in sensitivity. This modification effect is particularly marked for the case of an annealing time of 0 seconds, and amongst the different modified primers, the effect is particularly high for a primer to which a 12mer with GGAC as the repeating unit has been added.

With the modified primers, a practical level of amplification can be achieved even using annealing temperatures and annealing times for which amplification does not occur with the unmodified primer (refer to FIG. 4). This effect is also particularly marked for the primer to which a 12mer with GGAC as the repeating unit has been added.

The final quantity of the amplified product after 40 cycles is markedly higher for the modified primers than for the unmodified primer (refer to FIG. 1 through FIG. 4). In normal PCR, because the amplification reaction is not usually conducted beyond 40 cycles, the modified primers offer a distinct advantage within a practical cycle range.

The above results reveal a modification effect under conditions of both increased temperature and shortened annealing time, and it is thought that these effects are due to an improvement in the thermal stability of the hybridization between the primer and the template DNA, that is, an increase in the bonding strength.

According to the first aspect of the present invention, preliminary tests for investigating the annealing conditions and the like can be simplified considerably, and the PCR amplification efficiency can be improved. These effects are particularly marked in those cases in which the PCR is either asymmetric PCR or degenerate PCR.

Furthermore, according to the second aspect of the present invention, the hybridization specificity of an oligonucleotide to a DNA sample can be improved.

## Claims

1. A process for improving efficiency of a DNA amplification reaction, wherein a primer, in which a compound selected from a group consisting of LC-Red 705, an amino group, a phosphate group, biotin, DIG, DNP, TAMRA, Texas-Red, ROX, XRITC, rhodamine, LC-Red 640, a mercapto group, psoralen, cholesterol, FITC, 6-FAM, TET, cy3, cy5, BODIPY 564/570, BODIPY 500/510, BODIPY 530/550, BODIPY 581/591 and oligonucleotides with a combined G and C content of at least 25% and with at least four bases is added to a 5' terminus, is used as a primer.

2. A process for improving efficiency of a DNA amplification reaction according to claim 1, wherein said oligonucleotide with a combined G and C content of at least 25% and with at least four bases has a combined G and C content of at least 50%, comprises no more than 40 bases, and has a quantity of a more numerous base of G and C that accounts for at least 50% of said combined G and C content, and a quantity of a more numerous base of A and T that accounts for at least 50% of a combined content of A and T.

3. A process for improving efficiency of a DNA amplification reaction according to either one of claim 1 and claim 2, which is a process for improving PCR amplification efficiency.

4. A process for improving efficiency of a DNA amplification reaction according to claim 3, wherein said PCR is either one of asymmetric PCR and degenerate PCR.

5. A process for improving hybridization specificity of an oligonucleotide to a DNA, wherein an oligonucleotide in which a compound selected from a group consisting of LC-Red 705, an amino group, a phosphate group, biotin, DIG, DNP, TAMRA, Texas-Red, ROX, XRITC, rhodamine, LC-Red 640, a mercapto group, psoralen, cholesterol, FITC, 6-FAM, TET, cy3, cy5, BODIPY 564/570, BODIPY 500/510, BODIPY 530/550 and BODIPY 581/591 is conjugated to a 5' terminus is used for hybridizing to said DNA.


SEQUENCE LISTING

<110>  Nichirei Corporation

<120>  Process for improving efficiency of DNA amplification re
action

<130>  3J33105-2EP

<160>  19

<170>  PatentIn version 3.1

<210>  1
<211>  20
<212>  DNA
<213>  primer

<400>  1
aagaagacct agaagatgat

20

<210>  2
<211>  18
<212>  DNA
<213>  primer

<400>  2
gttaccagta atagggca

18

<210>  3
<211>  21
<212>  DNA
<213>  primer

<220>
<221>  misc_feature
<222>  (7)..(7)
<223>  n is A, C, G, T or U

<220>
<221>  misc_feature
<222>  (16)..(16)
<223>  n is A, C, G, T or U

<220>

<221>  misc_feature
<222>  (19)..(19)
<223>  n is A, C, G, T or U


<400>  3
yatgmgngar atgggnacng t

21


<210>  4
<211>  21
<212>  DNA
<213>  primer

<220>
<221>  misc_feature
<222>  (1)..(1)
<223>  n is A, C, G, T or U


<220>
<221>  misc_feature
<222>  (7)..(7)
<223>  n is A, C, G, T or U


<400>  4
ngcytcnacc atytcyttyt t

21


<210>  5
<211>  12
<212>  DNA
<213>  primer

<400>  5
agtcagtcag tc

12


<210>  6
<211>  12
<212>  DNA
<213>  primer

<400>  6
aagtaagtaa gt

12

<210> 7
<211> 12
<212> DNA
<213> primer

<400> 7
ggacggacgg ac

12

<210> 8
<211> 12
<212> DNA
<213> primer

<400> 8
gggcgggcgg gc

12

<210> 9
<211> 12
<212> DNA
<213> primer

<400> 9
aaataaataa at

12

<210> 10
<211> 16
<212> DNA
<213> primer

<400> 10
agtcagtcag tcagtc

16

<210> 11
<211> 16
<212> DNA
<213> primer

<400> 11

aagtaagtaa gtaagt

16

<210> 12
<211> 16
<212> DNA
<213> primer

<400> 12
ggacggacgg acggac

16

<210> 13
<211> 16
<212> DNA
<213> primer

<400> 13
gggcgggcgg gcgggc

16

<210> 14
<211> 16
<212> DNA
<213> primer

<400> 14
aaataaataa ataaat

16

<210> 15
<211> 20
<212> DNA
<213> primer

<400> 15
agtcagtcag tcagtcagtc

20

<210> 16
<211> 20
<212> DNA
<213> primer

<400>  16
aagtaagtaa gtaagtaagt

20

<210>  17
<211>  20
<212>  DNA
<213>  primer

<400>  17
ggacggacgg acggacggac

20

<210>  18
<211>  20
<212>  DNA
<213>  primer

<400>  18
gggcgggcgg gcgggcgggc

20

<210>  19
<211>  20
<212>  DNA
<213>  primer

<400>  19
aaataaataa ataaataaat

20